# EUROPÄISCHE PATENTSCHRIFT

(11) **EP 0 338 975 B1**
(45) Veröffentlichungstag und Bekanntmachung des Hinweises auf die Patenterteilung: **10.03.1993**
(21) Anmeldenummer: 89810213.2
(22) Anmeldetag: 17.03.1989
(51) Int. Cl.: A61B 3/10, A61F 9/00

(54) **Gerät zur Untersuchung und Behandlung des Auges**
Ophthalmic apparatus for performing examination and therapy
Appareil ophtalmologique pour l'examen et le traitement

(30) Priorität: 20.04.1988 CH 1455/88
(43) Veröffentlichungstag der Anmeldung: 25.10.1989
(73) Patentinhaber: HAAG-STREIT AG Werkstätten für Präzisionsmechanik, CH-3098 Köniz (CH)
(72) Erfinder: Papritz, Stephan, CH-3098 Schliern (CH); Schröder, Eckhard, D-8501 Eckental (DE)
(74) Vertreter: Steiner, Martin

(56) Entgegenhaltungen:
- EP-A- 0 030 120
- EP-A- 0 225 699
- WO-A-87/05495

## Beschreibung

Die vorliegende Erfindung betrifft ein Gerät zur Untersuchung und Behandlung des Auges, mit Mitteln zur Beleuchtung des Auges und Mitteln zum Einkoppeln eines Laserstrahls in die Beleuchtungsoptik zur Photokoagulation. Derartige Geräte, insbesondere Spaltlampen, sind bekannt.

Bei einer bekannten Ausführung befindet sich im Beleuchtungstubus ein halbtransparenter Spiegel, durch welchen das Beleuchtungslicht durchtritt und welcher den eingekoppelten Laserstrahl in die Achse der Beleuchtungsoptik ablenkt (DE-A-2 611 933). Diese zwar im Aufbau einfache Lösung bringt erhebliche Lichtverluste sowohl für die Beleuchtung als auch für den Laserstrahl mit sich.

Um diese Nachteile mindestens teilweise zu vermeiden, ist es auch bekannt, im Beleuchtungstubus unmittelbar nach dem Kondensor ein Prismenpaar vorzusehen, mittels welchem das Beleuchtungslicht in zwei seitliche Teilstrahlen aufgeteilt wird, welche seitlich des der Einkoppelung des Laserstrahls dienenden Spiegels liegen. Der Laserstrahl und die beiden Teilstrahlenbündel der Beleuchtung werden sodann durch gesonderte Spiegel ins Auge geworfen (EP-A-0 225 699). Auch diese Ausführung hat jedoch verschiedene Nachteile. Es bedarf einer speziellen Ausführung des Beleuchtungstubus mit zusätzlichen Prismen, und dieser Beleuchtungstubus muss einen grösseren Durchmesser aufweisen. Es kann somit nicht der Beleuchtungstubus einer bestehenden diagnostischen Spaltlampe verwendet werden. Die heute allgemein übliche und erwünschte Drehung der Spaltblende bzw. des ganzen Beleuchtungstubus mit der Spaltblende ist nicht oder nur in beschränktem Masse möglich, weil bei grösserer Drehung um bis zu 90° kein Licht mehr über die Umlenkspiegel ins Auge gelangen würde.

Der vorliegenden Erfindung liegt die Aufgabe zugrunde, die oben erwähnten Nachteile zu vermeiden und zusätzliche Vorteile zu erzielen. Die erfindungsgemässe Lösung dieser Aufgabe ist im Kennzeichen des Anspruchs 1 umschrieben. Unter diesen Umständen kann nun ein herkömmlicher Beleuchtungstubus ohne jede Aenderung verwendet werden. Es zeigt sich ferner, dass man die Spaltblende bzw. den ganzen Beleuchtungstubus mit der Spaltblende um bis zu 90° drehen kann, ohne wesentlich an Helligkeit des Spaltbildes einzubüssen. Schliesslich zeigt es sich, dass man bei einer bestimmten ausgeleuchteten Fläche im Beleuchtungsobjektiv verglichen mit der Ausführung gemäss EP-A-0 225 699 ein doppelt so helles Spaltbild erzielt. Es ergeben sich somit insgesamt überraschend viele Vorteile. Die Erfindung wird nun anhand eines in der Zeichnung dargestellten Ausführungsbeispiels und einiger Varianten näher erläutert.
Fig. 1 ist eine schematische Darstellung des Ausführungsbeispiels,
Fig. 2 bis 4 zeigen die Querschnitte der Teilstrahlenbündel einer Spaltbeleuchtung im Frontobjektiv, und
Fig. 5 bis 9 zeigen Ausführungsvarianten.

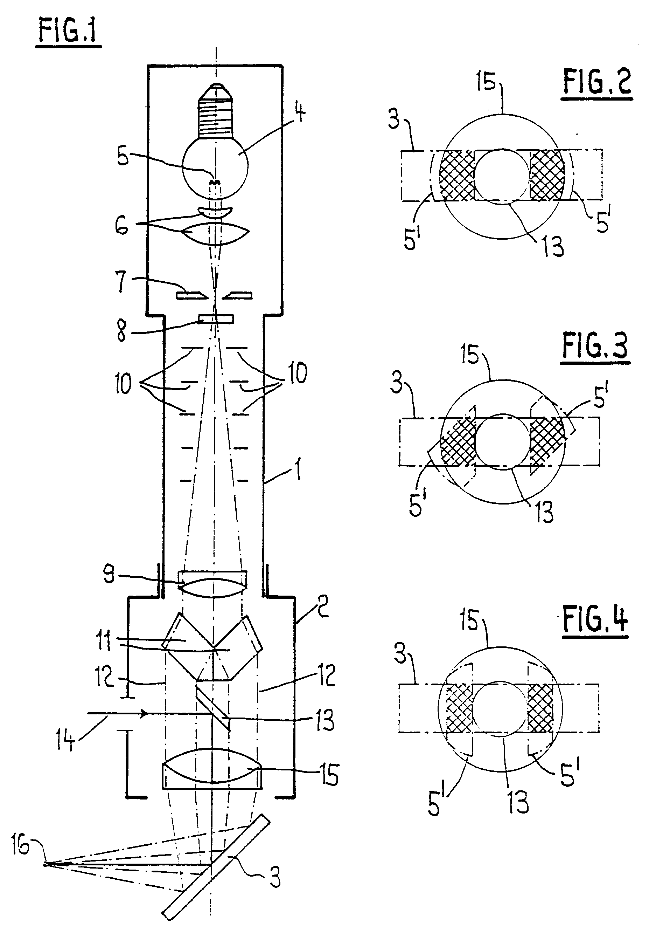

Fig. 1 zeigt schematisch einen Beleuchtungstubus 1, der um seine Vertikalachse drehbar in einem Tubus 2 gelagert ist. Unterhalb des Tubus 2 ist ein Beleuchtungsspiegel 3 angeordnet. Der Tubus 2 und der Beleuchtungsspiegel 3 sind in starrer gegenseitiger Lage auf einem nicht dargestellten Schwenkarm der Spaltlampe angeordnet. Im Beleuchtungstubus 1 ist eine Glühlampe 4 mit einer Glühwendel 5 angeordnet, wobei die Achse der Glühwendel 5 in der Zeichnungsebene liegt. Unterhalb der Glühlampe befinden sich ein Kondensor 6, eine Spaltblende 7, ein Filter 8 und ein Tubusobjektiv 9. Zwischen dem Filter 8 und dem Tubusobjektiv 9, welches die Spaltblende nach Unendlich abbildet, befinden sich im Beleuchtungstubus 1 Kulissenblenden 10 zur Herabsetzung von Streulicht im Beleuchtungstubus. Diese Ausführung des Beleuchtungstubus entspricht der für Spaltlampen üblichen.

Unterhalb des Tubusobjektivs 9 befindet sich im Tubus 2 ein Reduktionsprismenpaar 11. Wie in Fig. 1 gezeigt, zerlegt dieses Prismenpaar das aus dem Tubusobjektiv austretende Bündel paralleler Strahlen in zwei nach aussen versetzte Teilstrahlenbündel 12. Diese Strahlenbündel liegen seitlich ausserhalb eines in der Achsprojektion kreisrunden Einkoppelungsspiegels 13 für einen quer in den Tubus 2 eingeführten Laserstrahl 14. Dieser Laserstrahl sowie die Teilstrahlenbündel 12 treten durch ein Frontobjektiv 15 auf den Beleuchtungsspiegel 3, welcher sie zum Spaltbild bzw. Laserbehandlungsfleck an der Stelle 16 umlenkt.

Aus Fig. 1 ist klar ersichtlich, dass der Beleuchtungsstrahlengang durch den Spiegel 13 zur Einkoppelung des Laserstrahles 14 nicht behindert oder beschnitten wird. Es findet also keinerlei gegenseitige Benachteiligung des Beleuchtungssystems und der Einkoppelung des Laserstrahls und insbesondere kein Lichtverlust in den beiden Systemen statt. Trotzdem der Beleuchtungsspiegel 3 verhältnismässig schmal ausgeführt sein muss, um den Strahlengang des nicht dargestellten Stereomikroskops der Spaltlampe nicht zu behindern, ergibt sich für alle Drehstellungen der Spaltblende 7 in überraschender Weise ein genügend helles Spaltbild. Die Verhältnisse sind in den Figuren 2 bis 4 schematisch dargestellt. Mit 3 ist dort der verhältnismässig schmale Beleuchtungsspiegel 3 angedeutet. Mit 15 ist die Oeffnung des Frontobjektivs bezeichnet, 13 bedeutet den Durchmesser des Einkoppelungsspiegels 13 bzw. des Laserstrahls im Frontobjektiv, und mit 5′ sind die Teilbilder der Glühwendel 5 bezeichnet. Aus Fig. 2 ist hierbei ersichtlich, dass bei parallel zur Längsrichtung des Spiegels 3 stehender Glühwendel (gemäss Fig. 1) der durch die schraffierten Flächen bezeichnete Querschnitt der Beleuchtungs-Strahlenbündel eine optimale Grösse aufweist.

Fig. 3 zeigt die Verhältnisse bei um 45° gedrehtem Beleuchtungstubus und entsprechend gedrehter Spaltblende 7 und Glühwendel 5. Die Teilbilder der Glühwendel sind durch die Prismen 11 nach aussen versetzt, aber um 45° geneigt. Man sieht, dass die Querschnitte der beiden Beleuchtungs-Strahlenbündel nur unerheblich geringere Flächen aufweisen als bei optimaler Ausbeute gemäss Fig. 2.

Fig. 4 zeigt die Verhältnisse bei um 90° gedrehtem Beleuchtungstubus 1 und entsprechend gedrehter Spaltblende 7 bzw. Glühwendel 5. Die Teilbilder der Glühwendel 5′ stehen nun quer zur Längsachse des Spiegels 3, und sie sind durch die Prismen 11 nach aussen versetzt. Hierbei betragen die Querschnitte der Beleuchtungs-Strahlenbündel noch mindestens die Hälfte der Querschnitte bei optimaler Stellung gemäss Figur 2. Das Spaltbild ist also noch mindestens halb so hell wie bei optimaler Stellung, was praktisch kaum erkennbar und jedenfalls nicht nachteilig ist.

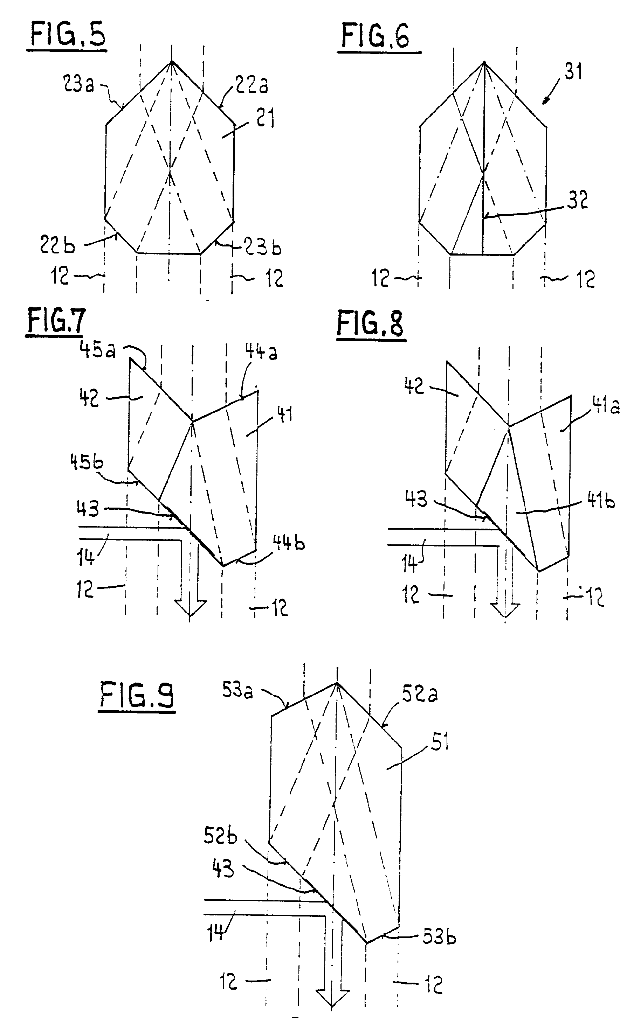

Die Fig. 5 bis 9 zeigen Ausführungsvarianten der Optik zum Aufweiten des Beleuchtungs-Strahlengangs. Gemäss Fig. 5 ist ein einteiliges Pisma 21 mit Paaren von parallelen Flächen 22a, 22b bzw. 23a, 23b vorgesehen, welches das eintretende Strahlenbündel in zwei sich überkreuzende und mit Abstand voneinander austretende Teilstrahlenbündel 12 aufteilt.

Fig. 6 zeigt eine aus zwei symmetrischen Einzelprismen bestehende Optik 31, deren Form äusserlich derjenigen des Prismas 21 nach Fig. 5 entspricht. Die Stossflächen 32 der beiden Prismen sind verspiegelt, sodass die am Eintritt einwärts gebrochenen Teilstrahlenbündel reflektiert werden und sich somit nicht überkreuzen, sondern nur in sich umgekehrt werden.

Die Fig. 7 bis 9 zeigen Ausführunsvarianten der Optik, bei welchen die Prismen zur Aufweitung des Beleuchtungs-Strahlengangs kombiniert sind bzw. als Träger dienen für den Spiegel zur Einkoppelung des Laserstrahls. Gemäss Fig. 7 sind zwei Prismen 41 und 42 miteinander verbunden, die im wesentlichen gemäss Fig. 1 die parallelen Teilstrahlenbündel 12 erzeugen. Das Prisma 41 weist eine verspiegelte Fläche 43 auf, die der Umlenkung des eintretenden Laserstrahls 14 dient. Bedingt durch die geneigte verspiegelte Fläche 43 muss das Prisma 41 länger sein als das Prisma 42. Die brechenden Flächen 44a und 44b am Eintritt und Austritt weisen daher geringere Neigungen auf als die brechenden Flächen 45a und 45b des Prismas 42, um eine symmetrische Aufweitung des Beleuchtungs-Strahlenbündels zu erzielen.

Fig. 8 zeigt eine ähnliche Optik wie Fig. 7, wobei jedoch das Prisma 41 in zwei Prismen 41a und 41b aufgeteilt ist. Der keilförmige Prismenteil 41b hat keine Funktion zur Aufweitung des Beleuchtungs-Strahlenbündels, sondern dient mit seiner verspiegelten Fläche 43 der Umlenkung des Laserstrahls 14.

Fig. 9 zeigt ein einteiliges Prisma 51 ähnlich demjenigen nach Fig. 5, jedoch asymmetrisch gestaltet, um mit einer verspiegelten Fläche 43 der Umlenkung des Laserstrahls 14 dienen zu können. Auch in diesem Falle weisen die brechenden Flächenpaare 52, 52b und 53, 53b ungleiche Neigungen auf, um trotz der unterschiedlichen optischen Längen eine symmetrische Aufweitung oder Aufspreizung in zwei Teilstrahlenbündel 12 zu erzielen.

Bei den Ausführungsvarianten nach Fig. 7 bis 9 sind unterschiedlich geneigte brechende Flächenpaare vorgesehen, um eine symmetrische Aufweitung in zwei Teilstrahlenbündel 12 zu erzielen. Es wäre jedoch auch möglich, gleich geneigte brechende Flächenpaare vorzusehen und damit eine Aufweitung in zwei zur optischen Achse des Tubus 1 asymmetrische Teilstrahlenbündel 12 vorzunehmen. Es wäre auch möglich, nur einen Teil des aus dem Objektiv 9 austretenden Strahlenbündels nach aussen zu versetzen und den anderen Teil unversetzt durchtreten zu lassen. Hierbei wäre die verspiegelte Fläche 43 und der Eintritt des Laserstrahls 14 so zu legen, dass der umgelenkte Laserstrahl wieder symmetrisch zwischen den beiden Teilstrahlenbündeln 12 liegt. Das Frontobjektiv wäre ebenfalls symmetrisch zu den Teilstrahlenbündeln 12 und zum Laserstrahl 14, also mit einer gegenüber der optischen Achse des Tubus 1 versetzten Achse anzuordnen.

Bei den Ausfürungen nach Fig. 1, 5 und 6 könnten die Prismen um 90° drehbar sein, um wahlweise mit einem Spiegel 3 zusammenzuwirken, dessen grössere Abmessung entweder gemäss Ausführungsbeispiel parallel oder aber quer zur Zeichnungsebene liegt. In vielen Fällen ist die ganze Spaltbeleuchtung gegenüber dem Spiegel 3 um eine senkrecht zur Zeichnungsebene in Fig. 1 stehende Achse neigbar, und in diesem Falle könnte der Spiegel 3 T-förmig, oben schmal und unten breit ausgeführt werden. Je nach der Neigung der Spaltbeleuchtung könnten die Prismen entsprechend eingestellt werden, um das Strahlenbündel in der Zeichnungsebene (Fig. 1) oder quer zu derselben aufzuweiten.

## Patentansprüche

1. Spaltlampe mit einem eine Lichtquelle (4,5), eine Spaltblende (7) und ein Tubusobjektiv (9) aufweisenden Beleuchtungstubus (1), der in einem weiteren Tubus (2) drehbar angeordnet ist, um die Spaltbeleuchtung in einem Bereich von 90° zu drehen, und mit einem Frontobjektiv (15) sowie einem Beleuchtungs-Umlenkspiegel (3) ausserhalb des weiteren Tubus (2), dadurch gekennzeichnet, dass sich im weiteren Tubus (2) zwischen dem Tubusobjektiv (9) und dem Frontobjektiv (15) eine Optik (11,21,31,41,42,51) und ein voll reflektierender Laser-Umlenkspiegel (13) für einen einzukoppelnden Laserstrahl (14) befinden, wobei die Optik den vollen Beleuchtungsstrahlengang (12) aufweitet und seitlich des Laser-Umlenkspiegels vorbeiführt, und wobei das Frontobjektiv (15) für den Beleuchtungsstrahlengang und den Laserstrahl gemeinsam ist.

2. Spaltlampe nach Anspruch 1, dadurch gekennzeichnet, dass die Optik (11) zum Aufweiten des Beleuchtungs-Strahlengangs ein Prismenpaar (11) aufweist.

3. Spaltlampe nach Anspruch 2, dadurch gekennzeichnet, dass die Prismen (11) zur optischen Achse entgegengesetzt geneigt sind und je zwei planparallele, optisch aktive Flächen aufweisen.

4. Spaltlampe nach einem der Ansprüche 1 bis 3, dadurch gekennzeichnet, dass die Optik (11,21,31,41,42,51) um die Achse der Beleuchtungsoptik drehbar ist.

5. Spaltlampe nach einem der Ansprüche 1 bis 4, dadurch gekennzeichnet, dass die Optik (41,42, bzw. 51) eine verspiegelte Fläche (43) aufweist, welche der Umlenkung bzw. Einkoppelung des Laserstrahls (14) dient.

6. Spaltlampe nach Anspruch 5, dadurch gekennzeichnet, dass die Optik asymmetrisch geformte bwz. angeordnete Prismen (41,42,51) mit unterschiedlich geneigten brechenden parallelen Flächenpaaren (44,45 bzw. 52,53) zur Erzielung einer symmetrischen Aufweitung des Beleuchtungs-Strahlengangs aufweist.

7. Spaltlampe nach Anspruch 5, dadurch gekennzeichnet, dass die Optik asymmetrisch geformte bzw. angeordnete Prismen mit gleich geneigten brechenden Flächenpaaren zur Erzielung einer asymmetrischen Aufweitung des Beleuchtungs-Strahlengangs aufweist.

## Claims

1. Slit lamp with a lighting tube (1) comprising a luminous source (4,5), a slit diaphragm (7) and a tube objective (9), and which is pivotably arranged in an other tube (2) in order to permit the rotation of the slit lighting in a range of 90°, and with a frontal objective (15) as well as a lighting deviation mirror (3) outside of the other tube (2), characterized in that in the tube (2), between the tube objective (9) and the front objective (15), there is provided an optic (11,21,31,41,42,51) and a laser deviation mirror having a total reflection (13) for a laser ray (14) to be coupled, the optic widening the total path of the lighting ray (12) and passing lateraly to the laser deviation mirror and the front objective (15) being common to the path of the lignting ray and to the laser ray.

2. Slit lamp according to claim 1, characterized in that the optic (11) comprises a pair of prisms (11) for widening the path of the lighting ray.

3. Slit lamp according to claim 2, characterized in that the prisms (11) are inclined in the opposite direction of the optical axis and that they comprise each two plane parallel optically active surfaces.

4. Slit lamp according to one of the claims 1 to 3, characterized in that the optic (11,21,31,41,42,51) is pivotably arranged about the axis of the lighting optic.

5. Slit lamp according to one of the claims 1 to 4, characterized in that the optic (41,42, resp. 51) comprises a reflecting surface (43) which serves to the deviation, resp. the coupling of the laser ray (14).

6. Slit lamp according to claim 5, characterized in that the optic comprises prisms (41,42,51) of asymmetrical form, resp. asymmetrically arranged, with pairs of parallel refringent, diversely inclined surfaces (44, 45,resp. 52,53) in order to optain a symmetrical widening of the path of the lignting ray.

7. Slit lamp according to claim 5, characterized in that the optic comprises prisms of asymmetrical form, resp. asymmetrically arranged, with pairs of refringent surfaces equally inclinate in order to obtain an asymmetrical widening of the path of the lignting ray.

## Revendications

1. Lampe à fente avec un tube d'éclairage (1) comprenant une source lumineuse (4,5), un diaphragme à fente (7) et un objectif de tube (9), et qui est disposé de manière tournante dans un autre tube (2), afin de permettre la rotation de l'éclairage de fente dans un domaine de 90°, et avec un objectif frontal (15) ainsi qu'un miroir de déviation d'éclairage (3) en dehors de l'autre tube (2), caractérisée en ce que dans le tube (2), entre l'objectif de tube (9) et l'objectif frontal (15), se trouve une optique (11,21,31,41,42,51) et un miroir de déviation laser à réflection totale (13) pour un rayon laser (14) à coupler, l'optique élargissant le chemin total du rayon d'éclairage (12) et passant latéralement au miroir de déviation laser, et l'objectif frontal (15) étant commun au chemin du rayon d'éclairage et au rayon laser.

2. Lampe à fente selon la revendication 1, caractérisée en ce que l'optique (11) comprend une paire de prismes (11) pour élargir le chemin du rayon d'éclairage.

3. Lampe à fente selon la revendication 2, caractérisée en ce que les prismes (11) sont inclinés à l'opposé de l'axe optique et qu'ils comprennent chacun deux surfaces planes parallèles optiquement actives.

4. Lampe à fente selon l'une des revendications 1 à 3, caractérisée en ce que l'optique (11,21,31,41,42,51) est pivotante autour de l'axe de l'optique d'éclairage.

5. Lampe à fente selon l'une des revendications 1 à 4, caractérisée en ce que l'optique (41,42, resp. 51) comprend une surface réfléchissante (43) qui sert à la déviation, resp. au couplage du rayon laser (14).

6. Lampe à fente selon la revendication 5, caractérisée en ce que l'optique comprend des prismes (41,42,51) de forme asymétrique, resp. disposés asymétriquement, avec des paires de surfaces (44,45, resp. 52,53) parallèles réfringentes inclinées diversement pour obtenir un élargissement symétrique du chemin du rayon d'éclairage.

7. Lampe à fente selon la revendication 5, caractérisée en ce que l'optique comprend des prismes de forme asymétrique, resp. disposés asymétriquement, avec des paires de surfaces réfringentes inclinées également pour obtenir un élargissement asymétrique du chemin du rayon d'éclairage.
